# EUROPEAN PATENT APPLICATION

(11) **EP 4 703 788 A1**
(43) Date of publication of application: **04.03.2026**
(21) Application number: 25197170.1
(22) Date of filing: 20.08.2025
(51) Int. Cl.: G02C 7/02, A61F 9/02, G02C 11/00, H04N 23/57

(54) **EYEWEAR LENS ASSEMBLY WITH INTEGRATED CAMERA**

(30) Priority: 30.08.2024 US 202463689385 P
(71) Applicant: Oakley, Inc., Foothill Ranch, CA 92610 (US)
(72) Inventor: HOLMAN, Nicolas, Foothill Ranch (US); COHEN, Benjamin, Foothill Ranch (US); GITAJN, Rachel, Foothill Ranch (US)
(74) Representative: Vossius & Partner Patentanwälte Rechtsanwälte mbB

(57) **Abstract**

Eyewear comprising an integrated camera module including a point-of-view camera is disclosed. The camera module can be connected to the eyewear lens and supported exclusively by the eyewear lens.

## Description

### CROSS-REFERENCE TO RELATED APPLICATIONS

This application claims the benefit of United States Provisional Application No. 63/689,385, filed August 30, 2024 and titled "EYEWEAR WITH INTEGRATED CAMERA," the disclosure of which is incorporated by reference herein in its entirety.

### BRIEF SUMMARY

In an aspect, an eyewear lens assembly for eyewear can include an eyewear lens and a camera module comprising a camera lens. The camera module can be connected to and supported solely by the eyewear lens. The eyewear lens assembly camera module can capture a point of view of a wearer. The eyewear lens assembly camera module can be positioned at a top portion of the lens substantially centered on a wearer's brow. The eye wear lens assembly can be used with a goggle. The eyewear lens assembly camera module can include a camera module housing. The camera module housing can include an aperture for the camera lens. The camera module can also include an image sensor disposed within the camera module housing.

In an aspect, the eyewear lens assembly can include a camera battery, a memory to store electronic data related to the image sensor, and a wireless transceiver to transmit and receive the electronic data related to the image sensor. The camera battery and wireless transceiver can be disposed inside the camera module housing. The camera battery and wireless transceiver can be disposed inside a second housing. The second housing can be supported solely by the eyewear lens. In an aspect, the camera module housing can include a first portion positioned on an interior portion of the eyewear lens and a second portion positioned on an exterior portion of the eyewear lens. In an aspect, the first portion can be detachably attached to the second portion.

In an aspect, the eyewear lens can be a unitary shield lens for eyewear. The camera module can be detachably attached to the eyewear lens. In an aspect, the camera module can be fixed to the eyewear lens.

In an aspect, at least a portion of the camera module housing can extend through an aperture in the eyewear lens. The aperture can form a channel in a top surface of the eyewear lens. In an aspect, an interior of the channel can include a protrusion to engage the camera module housing.

In an aspect, the camera module can be adjustable to alter a viewing angle of the camera lens.

In an aspect, eyewear can include a frame and a lens assembly supported by the frame. The lens assembly can include an eyewear lens and a camera module comprising a camera lens. The camera module can be connected to and supported solely by the eyewear lens.

In an aspect, the eyewear can be a goggle. In another aspect, the eyewear can include earstems connected to the frame for supporting the head of a wearer. In an aspect the lens assembly can be detachably attached to the frame. In another aspect, the eyewear can include a second eyewear lens to be supported by the frame after removing the lens assembly from the frame.

The camera module can include a camera module housing. The camera module housing can include an aperture for the camera lens. The camera module can include an image sensor disposed within the camera module housing.

In an aspect, the eyewear can include a camera battery, a memory to store electronic data related to the image sensor, and a wireless transceiver to transmit and receive the electronic data related to the image sensor. In an aspect, the camera battery and wireless transceiver can be disposed inside the camera module housing. In an aspect, the camera battery and wireless transceiver can be disposed inside a second housing. The second housing can be supported solely by the eyewear lens. In an aspect, the second housing can be supported by a goggle strap connected to the eyewear lens frame.

In an aspect, the eyewear lens can be a unitary shield lens for eyewear. In an aspect, the camera module can be detachably attached to the eyewear lens. In an aspect, at least a portion of the camera module housing can be positioned on an interior portion of the eyewear lens. In an aspect, the camera module housing can include a first portion positioned on an interior portion of the eyewear lens and a second portion positioned on an exterior portion of the eyewear lens. The first portion can be detachably attached to the second portion.

In an aspect, at least a portion of the camera module housing can extends through an aperture in the eyewear lens. In another aspect, the aperture can form a channel in a top surface of the eyewear lens. An interior of the channel can include a protrusion to engage the camera module housing. In a further aspect, the camera module can be adjustable to alter a viewing angle of the camera module. The camera module can capture a point of view of a wearer. In an aspect, the camera module can be positioned at a top portion of the lens substantially centered on a wearer's brow.

In a further aspect, a method for forming an eyewear lens assembly can include connecting a camera module including a camera lens and an image sensor to an eyewear lens such that the camera module is supported exclusively by the eyewear lens. In an aspect, connecting the camera module to the eyewear lens can include detachably attaching the camera module to the eyewear lens. In an aspect, connecting the camera module to the eyewear lens can include positioning a portion of the camera module in an aperture in the eyewear lens. In another aspect, the aperture can form a channel in a top surface of the eyewear lens. In an aspect, an interior of the channel can include a protrusion to engage the camera module.

### BRIEF DESCRIPTION OF THE FIGURES

The accompanying figures, which are incorporated herein and form a part of the specification, illustrate the present disclosure and, together with the description, further serve to explain the principals thereof and to enable a person skilled in the pertinent art to make and use the same. Aspects of the present disclosure are best understood from the following detailed description when read with the accompanying figures. It is noted that features may not be drawn to scale. In fact, the dimensions of the features may be arbitrarily increased or reduced for clarity of discussion. In the drawings:
FIG. 1 is a perspective view of an eyewear, according to an aspect.
FIG. 2 is a perspective view of an eyewear, according to an aspect.
FIG. 3 is a perspective view of a lens assembly, according to an aspect.
FIG. 4 is a perspective view of a lens assembly, according to an aspect.
FIG. 4A is a front view of the lens assembly of FIG. 4.
FIG. 5 is an assembly view of the lens assembly of FIG. 4.
FIG. 6 is a perspective view of a lens assembly, according to an aspect.
FIG. 7 is an assembly view of the lens assembly of FIG. 6.
FIG. 8 is an assembly view of an alternate lens assembly, according to an aspect.
FIG. 9 is a perspective view of an eyewear including the lens assembly of FIG. 8.
FIG. 10 is a perspective view of a lens assembly, according to an aspect.
FIG. 11 is an assembly view of the lens assembly of FIG. 10.
FIG. 12 is a side view of a camera module, according to an aspect.

### DETAILED DESCRIPTION

Some aspects will now be described with reference to the accompanying figures, wherein like numerals refer to like or similar elements throughout. Although several aspects, embodiments, examples and illustrations are disclosed below, it will be understood by those of ordinary skill in the art that the concepts described herein extend beyond the specifically disclosed aspects, embodiments, examples and illustrations, and can include other uses of the concepts and modifications and equivalents thereof. The terminology used in the description presented herein is not intended to be interpreted in any limited or restrictive manner simply because it is being used in conjunction with a detailed description of certain specific aspects. In addition, aspects of the eyewear can comprise several novel features and no single feature is solely responsible for its desirable attributes or is essential to practicing the concepts herein described.

The term "lens" as used herein is used to broadly refer to an optical component. For example, eyeglass/sunglass lenses, vision shields, visors, and the like are included in the term "lens" or "lens for eyewear." The term "non-corrective" as used herein indicates a lack of optical power as understood for prescription lenses.

Spatially relative terms, such as "beneath," "underlying," "underneath," "below," "lower," "above," "over," "upper," "lower," and the like may be used herein for ease of description to describe one element or feature's relationship to another element(s) or feature(s) as illustrated in the figures. The spatially relative terms are intended to encompass different orientations of the device in use or operation in addition to the orientation depicted in the figures. The device may be otherwise oriented (rotated 90 degrees or at other orientations) and the spatially relative descriptors used herein may likewise be interpreted accordingly.

It is noted that references in the specification to "one embodiment," "an embodiment," "an example embodiment," "exemplary," etc., indicate that the embodiment described may include a particular feature, structure, or characteristic, but every embodiment may not necessarily include the particular feature, structure, or characteristic. Moreover, such phrases do not necessarily refer to the same embodiment. Further, when a particular feature, structure or characteristic is described in connection with an embodiment, it would be within the knowledge of one skilled in the art to effect such feature, structure or characteristic in connection with other embodiments whether or not explicitly described.

It is to be understood that the phraseology or terminology herein is for the purpose of description and not of limitation, such that the terminology or phraseology of the present specification is to be interpreted by those skilled in relevant art(s) in light of the teachings herein.

In some embodiments, the terms "about" and "substantially" can indicate a value of a given quantity that varies within 5 % of the value (e.g., ±1 %, ±2 %, ±3 %, ±4 %, or ±5 % of the value).

The terms "wearer," "user," and the like as used herein may refer to a median user in general, a median user according to a demographic, or a user having physical dimensions conforming to a standard or a well-known database of human measurements. For example, a typical eyewear wearer may be one having physical dimensions that conform to European Standards (EN), American National Standards Institute (ANSI), or anthropometric surveys, among others.

Additionally, although particular embodiments may be disclosed or shown in the context of particular types of eyewear, such as unitary lens eyeglasses, dual lens eyeglasses, eyeglasses having partial, full, or no orbitals, goggles, sunglasses, eyewear with earstems, eyewear with partial earstems, eyewear without earstems, and the like, it is to be appreciated that embodiments of the present invention may be used in any type of headworn support. For example, lens embodiments may be integrated into or attached to an item of headgear, such as a bicycle, skateboarding, snow, flight, sport, or other type of helmet with a vision shield, a visor, a hat, a headband, face mask, balaclava, breaching shield, or any other any headworn article that may support one or more lenses in the wearer's field of view. In some embodiments, the lens may be detachable from the headworn article so that the lens may be removed or replaced without damaging the headworn article.

As used herein, the term "disposed," as used for example in "a first layer is disposed over a second layer," means that the first layer is either directly placed against the second layer's surface, or that the first layer is indirectly placed over the second layer's surface with at least a third layer in between.

As used herein, the term "coupled," as used for example in "a first layer is coupled to a second layer" means that the first layer is disposed over the second layer (as "disposed" is defined above), or that the first layer is integrated into the second layer.

It is to be understood that the frame of reference (e.g., the axes and planes) described herein are discussed in connection within standard contexts with a user's head in an upright vertical position, For example, an anatomical superior-inferior axis is generally referred to in connection with a vertical axis, the anatomical medio-lateral axis is generally referred to in connection with a horizontal plane. This can be measured, for example, on a standard headform such as, but not limited to, an Alderson headform, an EN168 headform, a CSA Z262.2-14 headform, or any other standard headform. However, it is also understood that the frame of reference described herein may be shifted in other contexts.

A lens assembly including a camera module for eyewear is disclosed herein. The camera module can include a camera lens, image sensor, and a camera housing so that the camera module can be detachably attached directly to an eyewear lens. In this aspect the camera module can be supported solely by the lens to form an integrated lens and camera module assembly, discussed herein as a lens assembly. Integrating the camera module directly on the lens allows for a standard eyewear lens to be replaced with the lens assembly, or vice versa, with existing eyewear, such as in a retrofit. Alternatively, eyewear can include a lens without a camera module and a second lens with a camera module such that either lens can be used with the eyewear. The aspects disclosed herein support adding image capture functionality to eyewear without requiring a new eyewear design to support the lens assembly including the lens and camera module.

In an aspect, the lens assembly including the camera module can be retrofit onto any eyewear, for example, eyewear that utilizes a removable lens. In some aspects, the camera module can be retrofit directly onto any eyewear lens so as to render that eyewear lens camera-enabled. According to the aspects discussed herein, the wearer does not need additional equipment to wear or hold a camera module. The camera module can include a point-of-view (POV) camera to capture a wearer's line of sight during an activity, for example, during a competition or athletic event. For example, utilizing a POV camera in the camera module can capture the view of the wearer and allow others to see what the wearer sees. In an aspect, the camera module can be positioned at an upper center portion of a lens, e.g., between the wearer's eyes, to capture a wearer's POV. In a further aspect the camera module can be oriented at an optimal angle to capture the wearer's POV. In another aspect, the camera module can include at least one lens and at least one image sensor. The camera module can also include a power supply (e.g., battery), memory, a processor, and a transmitter or transceiver to transfer and/or save image data captured by the image sensor. In another aspect, one or more of the power supply, memory, a processor, and a transmitter or transceiver can be positioned in a separate electronics module and can be electrically connected to the camera module. In an aspect, the eyewear can capture and transmit image data for broadcast during a competition or event to display the wearer's POV.

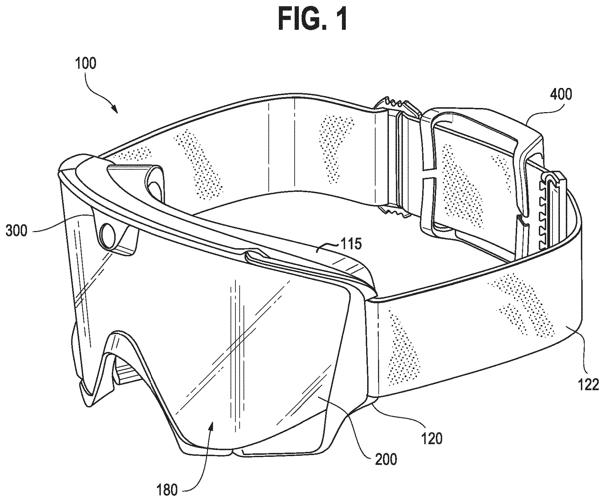

FIG. 1 shows an eyewear 100 including a lens assembly 180, a frame 120, strap 122, and an electronics module 400. Lens assembly 180 includes a lens 200 and a camera module 300.

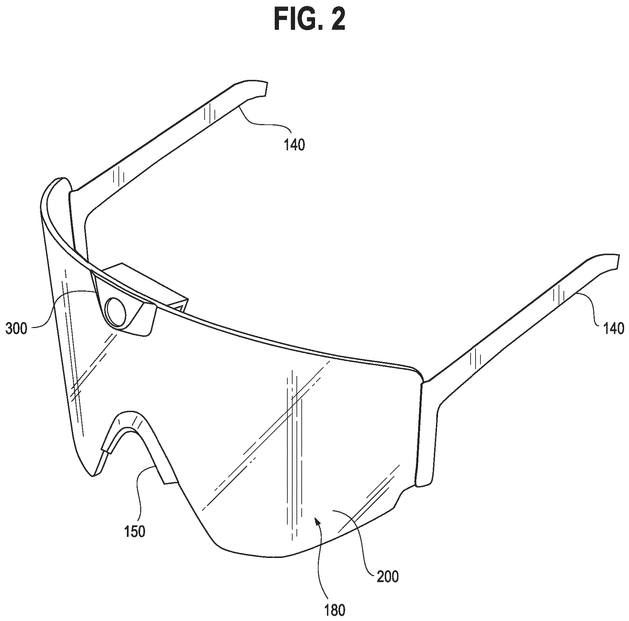

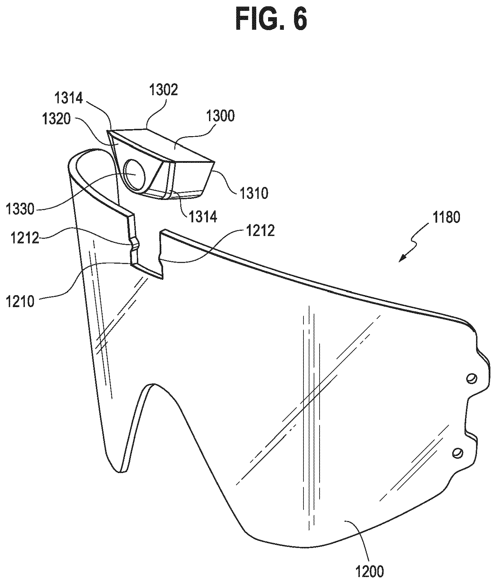

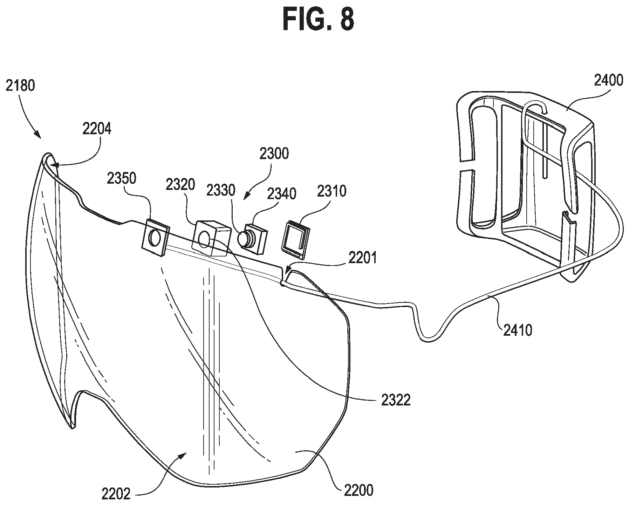

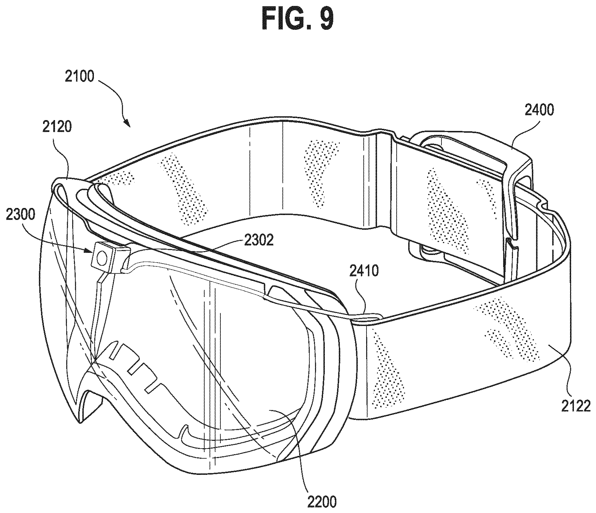

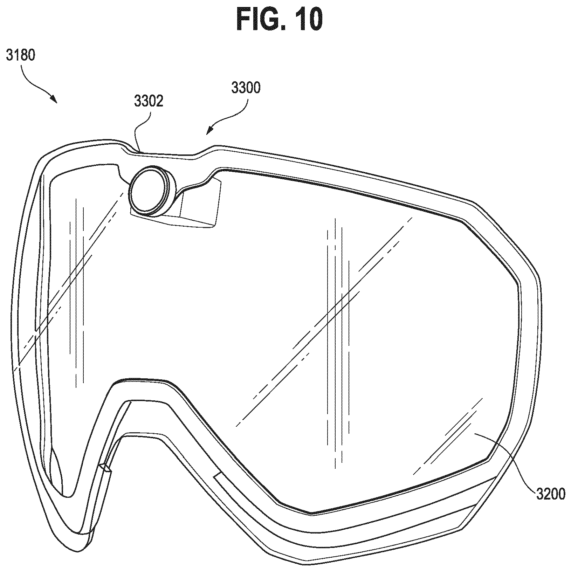

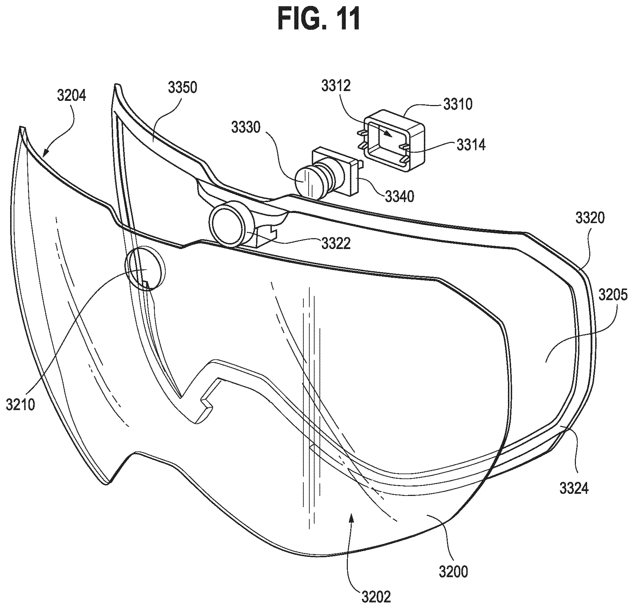

In an aspect, eyewear 100 can be a goggle, as shown for example in FIG. 1. In another aspect, eyewear 100 can be eyeglasses and include earstems 140 (FIG. 2) to support eyewear 100 on a head of a wearer. Eyewear 100 can be frameless with lens assembly 180 supported by earstems, as shown in FIG. 2, or supported by strap 122 (FIG. 1), or eyewear 100 can include frame 120 supporting lens assembly 180 and connected to strap 122 (FIG. 1) or earstems 140 for positioning lens 200 in the path of a wearer's field of vision. Eyewear 100 can also include a nosepiece 150. The embodiments of eyewear 100 shown in FIGS. 1-2 are only some examples of possible types of eyewear that lens assembly 180, lens assembly 1180 (FIGS. 6-7), lens assembly 2180 (FIGS. 8-9), and/or lens assembly 3180 (FIGS. 10-11) may be used in. By way of example and not limitation, lens assemblies 180, 1180, 2180, and 3180 can be incorporated into various types of eyewear, including, but not limited to, general-purpose eyewear, special-purpose eyewear, sunglasses, driving glasses, sporting glasses, goggles (including for sport or safety), visors, shields, indoor eyewear, outdoor eyewear, vision-correcting eyewear, prescription and non-prescription eyeglasses, color vision deficiency eyewear, contrast-enhancing eyewear, gaming eyewear, eyewear designed for another purpose (for example, helmet visors), or eyewear designed for a combination of purposes.

As shown in FIG. 1, eyewear 100 can be a goggle with lens assembly 180 and strap 122 attached to frame 120, for example at the left and right ends of lens assembly 180. Strap 122 can be attached to frame 120 such that it forms a loop configured to encircle the head of a user. Strap 122 may be constructed from a soft, elastic material and may include adjustment elements to adjust the length of strap 122. In some aspects, lens 200 can be designed with sufficient structural rigidity to serve as the main structural support of eyewear 100, and the eyewear can be frameless or frame 120 optionally included. Further, lens 200 may include attachment points for strap 122 and/or earstems 140. Examples of frameless eyewear are described in U.S. Patent No. 11,679,033 and U.S. Patent No. 11,526,025, which are both incorporated herein by reference in their entireties. In some aspects, the eyewear can be a visor attaching to headwear (e.g., eyewear is a helmet visor).

Lens 200 may be a unitary lens 200, such that a single lens 200 covers both eyes of the wearer. However, in some embodiments, lens 200 may be a dual lens with one lens covering each eye of the wearer. In some embodiments, lens 200 may include a single layer, or may include multiple layers. In some embodiments, lens 200 may be a laminated lens 200 formed by a series of stacked layers. In some embodiments, layers of lens 200 may include an outer layer and an inner layer that are separated by a space. The space may be filled with air or another gas to provide an air gap for thermal insulation.

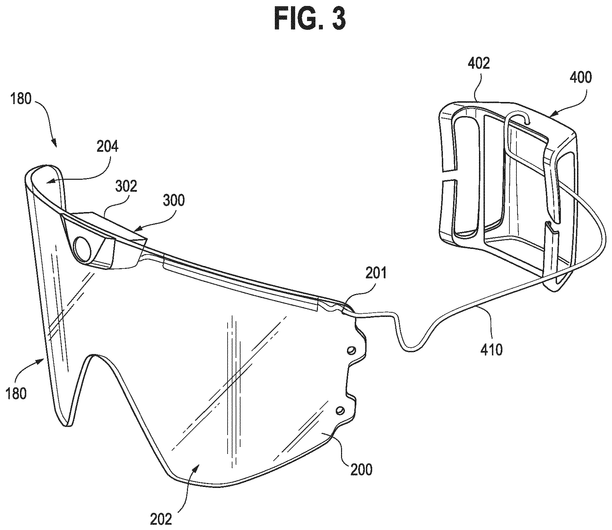

Lens 200 can include an outer surface 202 (FIG. 3) facing away from a wearer when eyewear 100 is worn, and an opposing inner surface facing toward a wearer's face when eyewear 100 is worn. In some embodiments, lens 200 may have a curvature, and the inner surface of lens 200 may have a concave curvature so as to contour to a shape of a wearer's face or head.

Lens 200 may be formed from any of various transparent materials, such as glass, for example a silicate glass, polycarbonate, polymethylmethacrylate, among other transparent materials. Lens 200 may be formed from a material that is strong, durable, and/or impact-resistant so that lens 200 does not readily crack or break and can withstand impact. In some embodiments, lens 200 may include a coating on an exterior surface and/or interior surface of lens 200, such as an anti-reflective coating, an anti-glare coating, or a UV-protective coating, among others.

In some embodiments, the lens assemblies 180, 1180, 2180, and/or 3180 may be movable. A goggle having a movable lens is described, for example, in U.S. Pat. Nos. 7,200,875 and 9,192,520, which are each incorporated herein by reference in their entireties. Lens 200 may be movably attached to frame 120 of the eyewear. In some embodiments, lens 200 is fully removable from frame 120 so that lens 200 may be replaced. Because lens 200 is removable, lens 200 may be interchanged with other lenses. Thus, lens 200 may be replaced without having to replace eyewear 100 in its entirety. Further, a wearer may desire to interchange lens 200 for another lens 200 having different properties. For example, a first lens 200 may be tinted or may have an anti-glare coating for use on bright days, while a second lens 200 may have multiple layers so as to provide improved thermal insulation. Additionally, a lens assembly 180 may be utilized such that a lens 200 can be replaced with a lens 200 having an integrated camera module 300. Further, a wearer may simply wish to interchange lenses 200 for aesthetic purposes to provide eyewear 100 with a desired appearance. Additionally, lens 200 may be removable such that the wearer may be able to remove lens 200 and/or lens assembly 180 without removing eyewear 100 from the wearer's head.

In some embodiments, eyewear 100 may include frame 120. Frame 120 may provide structural support to eyewear 100. Frame 120 may be shaped similarly to a perimeter of lens 200 when viewed from the front. In some aspects, frame 120 may be configured to secure lens 200. When lens 200 is secured to frame 120, interior surface 204 of lens 200 is in contact with frame 120 and maintains lens 200 in position in a wearer's field of view. In some embodiments, frame 120 may include various additional components of eyewear 100, such as one or more of outriggers for securing strap 122 to frame 120, a nose guard, and a locking assembly, among other components. Lens 200 may be spaced from a wearer's eyes and face so as to define a zone that is protected from moisture and particulate matter when eyewear 100 is worn. For example, face cushioning 115 (e.g., foam) may be supported on a rear side of the frame (or a rear side of lens assembly 180 in the aspect of a frameless eyewear) to space lens 200 from a wearer's eyes and face.

Frame 120 may be formed of a frame material that may include, for example, a nylon polymer, such as TR90 nylon. In some embodiments, frame material may be a high durometer rubber relative so that frame 120 can provide structural support to eyewear 100 and help eyewear 100 maintain its shape. Further, frame 120 may help to maintain proper lens curvature so as to preserve optics of eyewear 100.

As shown in FIGS. 1 and 3-4, lens assembly 180 can include camera module 300. In some aspects, lens assembly 180 can also include electronics module 400. Electronics module 400 can include one or more electronic components, and in an aspect, can be integral with camera module 300. In some aspects, the camera module 300 and electronics module 400 can be incorporated together into a single housing or in separate respective housings. As shown in FIG. 3, camera module 300 can be in a camera module housing 302, and electronics module 400 can be in an electronics module housing 402 with electronic component(s) consolidated together. In some aspects, eyewear 100 may include one or more electronic components separate from electronics module 400 and outside of electronics module housing 402 if included. In an aspect, electronics module 400 or separate electronic component(s) can be secured on location(s) or component(s) apart from lens 200. For example, electronics module 400 or separate electronic component(s) can be secured to strap 122 (FIG. 1) or attached to a helmet or other article of clothing (not shown). In an aspect, the electronic components of electronics module 400 or separate therefrom can include one or more of a power source, such as a battery, a memory (e.g., random access memory (RAM) and/or a hard disk drive and/or removable storage device or drive) to store image data, a transceiver or transmitter, a controller, and one or more sensors. In an aspect, the electronic components of electronics module 400 or separate therefrom can include sensors to detect one or more of an ambient temperature, a wearer's speed, a wearer's acceleration, a wearer's position, ambient light conditions, and/or other environmental conditions. For example, electronic components can include audio electronic components such as a speaker, a microphone, an environmental sensor such as a temperature sensor, a pressure sensor, an altitude sensor, an oxygen sensor, a light sensor and/or any other environmental sensor, a velocity sensor such as a fluid flow sensor or pitot tube, an accelerometer, a physiological sensor such as a heartbeat sensor, a blood-oxygen sensor, a perspiration sensor, an electrolyte sensor, a body temperature sensor, and/or any other sensor as desired, and/or a user interface. In another aspect, electronics components can include a global positioning system receiver to determine location of the eyewear at any time. Additionally or alternatively, electronics components may be configured to determine the eyewear's location using other satellite navigation systems (e.g., Galileo, BDS, GLONASS, etc.) or by using another means. Further, electronics components may include additional components (not shown) that enable the eyewear's location to be determined using other methods including WiFi positioning and/or cell tower triangulation.

In some aspects, electrical power can be provided by an onboard battery that is supported by or positioned within the camera module 300 and/or electronics module 400. External power sources can also be utilized. An external charger can be connected with a component of the eyewear, such as with one of the camera module 300 and electronics module 400, to supply external electrical power to be stored in an onboard battery for later use. In some embodiments, an external battery can be connected for extended use of the system. For example, in some uses of eyewear, such as during skiing or other activities, a battery can be stored in a helmet, backpack, utility belt, or other external location carried on the body, and placed in electrical connection with the camera module 300 and/or electronics module 400.

As shown in FIG. 3, electronics module 400 can be connected to a portion of eyewear 100 apart from lens 200. In another aspect, electronics module 400 can be supported partially or exclusively by lens 200. In an aspect, electronics module 400 can be connected to the camera module 300 by a wire 410. In an aspect, wire 410 can extend along interior surface 204 of lens 200 to connect camera module 300 to electronics module 400. In an aspect, wire 410 can extend from interior surface 204 to outer surface 202 through an aperture 201 in lens 200. Alternatively, wire 410 can extend along outer surface 202 of lens 200 to connect camera module 300 to electronics module 400. In another aspect (not shown), electronics module 400 can be integral with camera module 300.

In one or more embodiments, electronics module 400 is configured to communicate using multiple long-range and/or short-range wireless technologies including cellular networks, WiFi, Bluetooth^{®}, infrared, etc. In one or embodiments, electronics module 400 is configured to wirelessly transmit the image data from camera module 300 and/or data collected from the one or more sensors.

Similarly, electronics module 400 can be configured to receive commands wirelessly. These commands may include, for example, start or stop image capture.

As discussed herein, camera module 300 can include one or more camera lenses 330, and one or more image sensors 340 to capture image data. In an aspect, camera module 300 can capture still photographs of the POV of the wearer. In another aspect, camera module 300 can capture a video stream of the POV of the wearer and can store this image data in a memory and/or transmit the image data for distribution and/or display. In an aspect, lens assembly 180 can capture and transmit image data for broadcast during a competition or event. In an aspect, electronics module 400 can include a display to show the image data from the camera module 300.

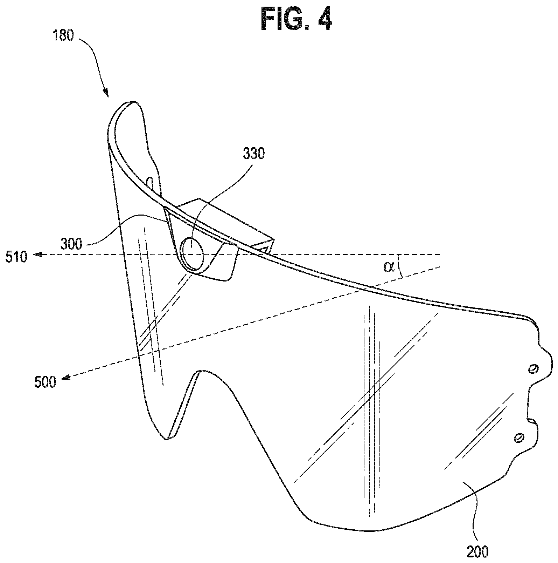

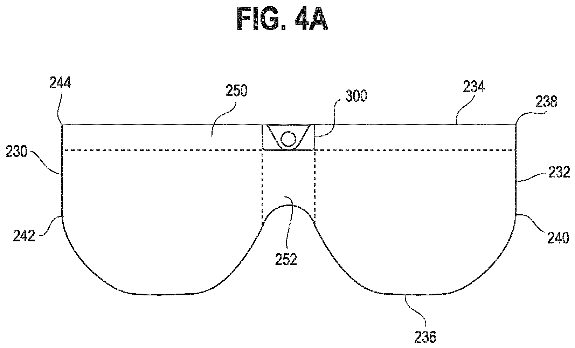

As shown in FIG. 4, camera lens 330 can be arranged at an optimal angle with respect to lens 200 to show the POV of the wearer. For example, camera lens 330 can be arranged along a camera axis 510 that corresponds to a camera line of sight that is positioned at an angle α with respect to wearer axis 500 that corresponds to a wearer line of sight. In an aspect, angle α can range from approximately 0 degrees to approximately 30 degrees. In another aspect, angle α can range from approximately 5 degrees to approximately 25 degrees, such as approximately 10 degrees to approximately 20 degrees, such as approximately 10 degrees to approximately 15 degrees, such as approximately 11 degrees.

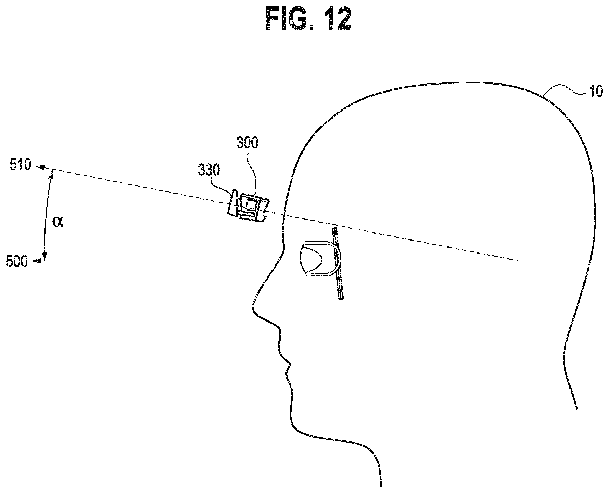

In an aspect, angle α can correspond to an angle of pantoscopic tilt with respect to wearer reference axis 500 corresponding to a line of sight of EN Headform 10 (FIG. 12). As discussed herein, a negative pantoscopic tilt corresponds to tilting upwards from reference axis 500. In this aspect, angle α can have a pantoscopic tilt in the range of approximately 0 degrees to approximately -30 degrees, such as approximately -5 degrees to approximately -25 degrees, such as approximately -10 degrees to approximately -20 degrees, such as approximately -10 degrees to approximately -15 degrees, such as approximately -11 degrees.

In an aspect, camera axis 510 can have a fixed orientation with respect to wearer axis 500. In another aspect, camera axis 510 can be movable with respect to wearer axis 500.

As discussed herein, camera module 300 can be connected to lens 200 such that the camera module 300 is supported exclusively by lens 200. In an aspect, camera module 300 can be detachably attached to lens 200. For example, camera module 300 can be detachably attached to lens 200 through a snap fit, snap hooks, a ratchet mechanism, or screws. In an alternate aspect, camera module 300 can be permanently fixed to lens 200, such as by attaching camera module 300 to lens 200 with an adhesive. As discussed herein, camera lens 330 (FIG. 4) can be positioned to protrude from outer surface 202 (FIG. 3) of lens 200. In another aspect, camera lens 330 can be positioned to be flush with the outer surface 202 of eyewear lens 200. In another aspect, camera lens 330, can be recessed in eyewear lens 200.

In an aspect, two or more camera modules 300 can be connected to lens 200 such that the camera modules 300 are supported exclusively by lens 200. In another aspect, eyewear 100 having a dual lens eyewear can include two camera modules 300, for example, a first camera module on a first lens 200 and a second camera module on a second lens 200. Utilizing two or more camera modules 300 with eyewear 100 can be utilized to provide stereoscopic, high definition, and/or three-dimension image data.

As shown in FIG. 4A, lens 200 can include a first side portion 230, a second side portion 232, a top portion 234, and a bottom portion 236. Lens 200 can also include a top portion and first side portion intersection 244, a top portion and second side portion intersection 238, a first side portion and bottom portion intersection 242, and a second side portion and bottom portion intersection 240. Lens 200 can also include an upper region 250 that is out of the wearer's forward line of sight. In an aspect, upper region 250 is positioned substantially out of the field of vision of the wearer to avoid obstruction the wearer's view. Lens 200 can also include a center region 252 that is between the wearer's eyes. In an aspect, camera module 300 can be positioned on lens 200 in upper region 250 and center region 252 to capture the wearer's POV without obstructing the wearer's vision. In this aspect, camera module 300 can be positioned at a top portion of lens 200 substantially centered at the wearer's brow laterally between wearer's eyes when eyewear 100 is worn.

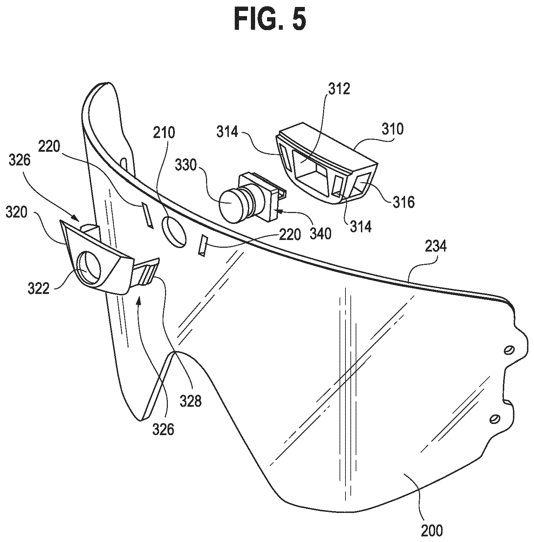

As shown in FIGS. 3-5, camera module 300 can include a camera module housing 302. The camera module housing 302 can include a front portion 320 and a back portion 310 such that the camera lens 330 and/or image sensor 340 can be secured within the camera module housing 302 and to the lens 200 when the front portion 320 and back portion 310 are connected. In an aspect, the back portion 310 of the camera module housing can be positioned on an interior surface 204 of lens 200. In another aspect, the front portion 320 of the camera module housing can be positioned on an outer surface 202 of lens 200. As shown in FIG. 5, the back portion 310 of the camera module housing can include a recess 312 to receive the image sensor 340 and camera lens 330. The front portion 320 can include an aperture 322 to receive camera lens 330. The front portion 320 of the camera module housing can also include one or more lock members 326. Lock members 326 can include a cantilever member 327 having a protrusion 328 to fit within a corresponding snap fit channel 314 and aperture 316 in back portion 310. Front portion 320 and back portion 310 can be disconnected by applying an inward force to cantilever member 327 through aperture 316 in back portion 310 and pulling front portion 320 and back portion 310 away from each other.

As shown in FIG. 5, one or more of lock members 326 can extend through a lock aperture 220 in lens 200. In an aspect, a first lock member 326 can extend through a first lock aperture 220 that extends through lens 200 and a second lock member 326 can extend through a second lock aperture 220 that extends through lens 200. In another aspect, one or more of lock members 326 can extend across top portion 234 of lens 200, without need for a lock aperature in lens 200.

In another aspect, lens 200 can include a camera lens aperture 210 so camera lens 330 can extend through lens 200 unobstructed. In this aspect, camera lens 330 can extend through camera lens aperture 210 and aperture 322 in front portion 320. In an aspect, camera lens aperture 210 can be positioned below top portion 234 so as not to interrupt the surface of top portion 234 of lens 200.

FIGS. 6-7 show an alternate lens assembly 1180. As shown, camera module 1300 can include a camera module housing 1302. The camera module housing 1302 can include a front portion 1320 and a back portion 1310 such that the camera lens 1330 and/or image sensor 1340 can be secured within the camera module housing 1302. In an aspect, camera module 1300 can be secured the lens 1200 by sliding camera module 1300 into camera lens aperture 1210. Camera lens aperture 1210 can be positioned on lens 1200 so camera lens 1330 can extend through lens 1200 unobstructed. In this aspect, camera lens 1330 can extend through camera lens aperture 1210 and aperture 1322 in front portion 1320. In an aspect, camera lens aperture 210 can be positioned in top portion 1234 such that camera lens aperture 1210 interrupts the surface of top portion 1234 of lens 1200 forming a channel therein. In an aspect, camera lens 1330 can be positioned to protrude from the outer surface of lens 1200. In another aspect, camera lens 1330 can be positioned to be flush with the outer surface of eyewear lens 1200. In another aspect, camera lens 1330, can be recessed in eyewear lens 1200.

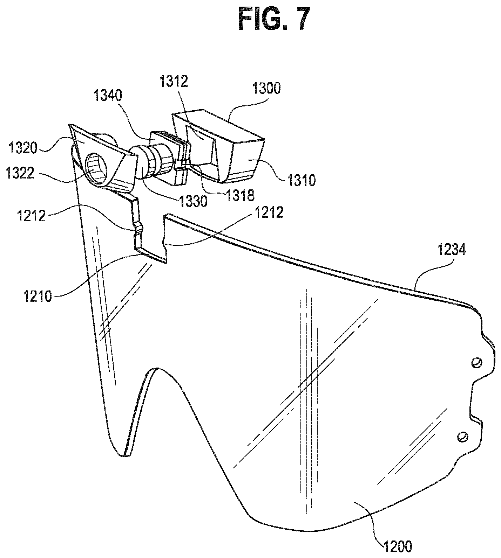

In an aspect, the back portion 1310 of the camera module housing can be positioned on an interior surface 1204 of lens 1200. In another aspect, the front portion 1320 of the camera module housing can be positioned on an outer surface 1202 of lens 200. As shown in FIG. 7, the back portion 1310 of the camera module housing can include a recess 1312 to receive the image sensor 1340 and camera lens 1330. The front portion 1320 can include an aperture 1322 to receive camera lens 1330. The front portion 1320 of the camera module housing can be connected to back portion 1310, for example by a snap fit or other attachment. When front portion 1320 is connected to back portion 1310, camera module housing 1302 can form a channel 1314 to engage lens 1200 by sliding camera module 1300 into camera lens aperture 1210. In an aspect, camera module housing 1302 can include a first channel 1314 to engage a first edge of camera lens aperture 1210 to connect camera module 1300 to lens 1200. In another aspect, camera module housing 1302 can include a second channel 1314 opposite first channel 1314 to engage a second edge of camera lens aperture 1210 opposite the first edge of camera lens aperture 1210 to connect camera module 1300 to lens 1200. In another aspect, one or more of the first edge or second edge of camera lens aperture 1210 can include a protrusion 1212 to form an interference fit with a corresponding channel 1314 on camera module 1300. In an aspect, one or more portions of camera module 1300 can include a notch 1318 to engage one or more protrusions 1212 and form an interference fit to secure camera module 1300 to lens 1200. For example, camera module housing 1302 can include a first notch 1318 to engage a first protrusion 1212 of camera lens aperture 1210 to connect camera module 1300 to lens 1200. In another aspect, camera module housing 1302 can include a second notch 1318 opposite the first notch 1318 to engage a second protrusion 1212 opposite the first protrusion 1212 of camera lens aperture 1210 to connect camera module 1300 to lens 1200.

Similar to the aspect shown in FIG. 3, a wire can connect camera module 1300 to an electronics module (not shown). In another aspect (not shown), the electronics module can be integral with camera module 1300.

FIGS. 8-9 show an alternate lens assembly 2180. As shown, eyewear 2100 can be a goggle with lens assembly 2180 and strap 2122 attached to frame 2120, for example at the left and right ends of lens assembly 2180. Strap 2122 can be attached to lens assembly 2180 such that it forms a loop configured to encircle the head of a user. Strap 2122 may be constructed from a soft, elastic material and may include adjustment elements to adjust the length of strap 2122. In some aspects, lens 2200 can be designed with sufficient structural rigidity to serve as the main structural support of eyewear 2100, and frame 2120 can be optionally included. Further, lens 2200 may include attachment points for strap 2122 and/or earstems 140.

As shown, camera module 2300 can include a camera module housing 2302. The camera module housing 2302 can include a front portion 2320 and a back portion 2310 such that the camera lens 2330 and/or image sensor 2340 can be secured within the camera module housing 2302. In an aspect, camera module 2300 can be secured to the lens 2200. In this aspect, front portion 2320 of camera module 2300 can be positioned on an interior surface 2204 of lens 2200. In an aspect, an adhesive film 2350 can be applied to front portion 2320 to adhere camera module 2300 to lens 2200. In this aspect, lens 2200 encloses camera lens 2330 and effectively serves as an outer lens to camera lens 2330.

As shown in FIG. 8, the back portion 2310 and the front portion 2320 of the camera module housing 2302 can receive the image sensor 2340 and camera lens 2330. The front portion 2320 can include an aperture 2322 to receive camera lens 1330. The front portion 2320 of the camera module housing can be connected to back portion 2310, for example by a snap fit or other attachment.

As shown, wire 2410 can extend along interior surface 2204 of lens 2200 to connect camera module 2300 to electronics module 2400. In an aspect, wire 2410 can extend from interior surface 2204 to outer surface 2202 through an aperture 2201 in lens 2200. In another aspect (not shown), electronics module 2400 can be integral with camera module 2300.

FIGS. 10-11 show an alternate lens assembly 3180 having an outer lens 3200 and an inner lens 3205. Outer lens 3200 can be separated from inner lens 3205 by a space that may be filled with air or another gas to provide an air gap for thermal insulation. Camera module 3300 can include a camera module housing 3302. The camera module housing 3302 can include a front portion 3320 and a back portion 3310 such that the camera lens 3330 and/or image sensor 3340 can be secured within the camera module housing 3302 and to the lens 3200. In an aspect, the front portion 3310 of the camera module housing can be positioned on an interior surface 3204 of outer lens 3200. As shown, the back portion 3310 of the camera module housing can include a recess 3312 to receive the image sensor 3340 and camera lens 3330. The front portion 3320 can include an aperture 3322 to receive camera lens 3330. The front portion can further include a front portion member 3324. In an aspect, front portion member 3324 can extend around a portion of the periphery of lens 3200 to surround a portion of the periphery of lens 3200. In another aspect, front portion member 3324 can extend around the entire periphery of lens 3200 to surround the periphery of outer lens 3200. In an aspect, front portion member 3324 can include a gasket to seal front portion member 3324 to inner lens 3205 and/or outer lens 3200. In an aspect, front portion 3320 including front portion member 3324 can be fixed to the interior surface 3204 of outer lens 3200 and/or inner lens 3205, for example using an adhesive. In an aspect, an adhesive film 3350 can be applied to front portion 3320 to adhere camera module 3300 to outer lens 3200.

The back portion 3310 of the camera module housing can also include one or more lock members 3314. Lock members 3314 can fit within a corresponding snap fit feature in front portion 3320.

In another aspect, lens 3200 can include a camera lens aperture 3210 so camera lens 3330 can extend through lens 3200 unobstructed. In this aspect, camera lens 3330 can extend through camera lens aperture 3210 and aperture 3322 in front portion 3320. In an aspect, camera lens aperture 3210 can be positioned below top portion 3234 so as not to interrupt the surface of top portion 3234 of lens 3200. In an aspect, camera lens 3330 can be positioned to protrude from the outer surface of lens 3200. In another aspect, camera lens 3330 can be positioned to be flush with the outer surface of eyewear lens 3200. In another aspect, camera lens 3330, can be recessed in eyewear lens 3200.

Numerical values, including endpoints of ranges, can be expressed herein as approximations preceded by the term "about," "substantially," "approximately," or the like. In such cases, other aspects include the particular numerical values. Regardless of whether a numerical value is expressed as an approximation, two aspects are included in this disclosure: one expressed as an approximation, and another not expressed as an approximation. It will be further understood that an endpoint of each range is significant both in relation to another endpoint, and independently of another endpoint.

The foregoing description of the specific aspects will so fully reveal the general nature of the aspects that others can, by applying knowledge within the skill of the art, readily modify and/or adapt for various applications such specific aspects, without undue experimentation, without departing from the general concept of the aspects. Therefore, such adaptations and modifications are intended to be within the meaning and range of equivalents of the disclosed aspects, based on the teaching and guidance presented herein. It is to be understood that the phraseology or terminology herein is for the purpose of description and not of limitation, such that the terminology or phraseology of the present specification is to be interpreted by those skilled in relevant art(s) in light of the teachings herein.

It is to be appreciated that the Detailed Description section, and not the Summary and Abstract sections, is intended to be used to interpret the claims. The Summary and Abstract sections may set forth one or more but not all exemplary aspects of the present disclosure as contemplated by the inventor(s), and thus, are not intended to limit the present disclosure and the appended claims in any way.

The breadth and scope of the aspects should not be limited by any of the above-described exemplary aspects, but should be defined only in accordance with the following claims and their equivalents.

The following aspects are preferred embodiments of the invention.
1. A eyewear lens assembly for eyewear comprising:
   an eyewear lens; and
   a camera module comprising a camera lens, the camera module being connected to and
   supported solely by the eyewear lens.
2. The eyewear lens assembly of aspect 1, wherein the camera module captures a point of view of a wearer.
3. The eyewear lens assembly of any preceding aspect, wherein the camera module is positioned at a top portion of the lens substantially centered on the lens.
4. The eyewear lens assembly of any preceding aspect, wherein the eyewear is a goggle.
5. The eyewear lens assembly of any preceding aspect, wherein the camera module comprises:
   a camera module housing including an aperture for the camera lens; and
   an image sensor disposed within the camera module housing.
6. The eyewear lens assembly of aspect 5, further comprising:
   a camera battery;
   a memory to store electronic data related to the image sensor; and
   a wireless transceiver to transmit and receive the electronic data related to the image sensor.
7. The eyewear lens assembly of aspect 6, wherein the camera battery and wireless transceiver are disposed inside the camera module housing.
8. The eyewear lens assembly of aspect 6 or aspect 7, wherein the camera battery and wireless transceiver are disposed inside a second housing.
9. The eyewear lens assembly of aspect 8, wherein the second housing is supported solely by the eyewear lens.
10. The eyewear lens assembly of any preceding aspect, wherein the eyewear lens is a unitary shield lens for eyewear.
11. The eyewear lens assembly of any preceding aspect, wherein the camera module is detachably attached to the eyewear lens.
12. The eyewear lens assembly of any preceding aspect, wherein the camera module is fixed to the eyewear lens.
13. The eyewear lens assembly of aspect 5, wherein at least a portion of the camera module housing is positioned on an interior portion of the eyewear lens.
14. The eyewear lens assembly of aspect 5 or aspect 13, wherein the camera module housing comprises a first portion positioned on an interior portion of the eyewear lens and a second portion positioned on an exterior portion of the eyewear lens.
15. The eyewear lens assembly of aspect 14, wherein the first portion is detachably attached to the second portion.
16. The eyewear lens assembly of aspect 5 or aspect 13, wherein at least a portion of the camera module housing extends through an aperture in the eyewear lens.
17. The eyewear lens assembly of aspect 16, wherein the aperture forms a channel in a top surface of the eyewear lens.
18. The eyewear lens assembly of aspect 17, wherein an interior of the channel includes a protrusion to engage the camera module housing.
19. The eyewear lens assembly of any preceding aspect, wherein the camera module is adjustable to alter a viewing angle of the camera lens.
20. Eyewear comprising:
   a frame; and
   a lens assembly supported by the frame, the lens assembly comprising:
      an eyewear lens; and
      a camera module comprising a camera lens, the camera module being connected to and supported solely by the eyewear lens.
21. The eyewear of aspect 20, wherein the eyewear is a goggle.
22. The eyewear of any of aspects 20-21, further comprising earstems connected to the frame for supporting the eyewear on a head of a wearer.
23. The eyewear of any of aspects 20-22, wherein the lens assembly is detachably attached to the frame.
24. The eyewear of any of aspects 20-23, further comprising a second eyewear lens to be supported by the frame after removing the lens assembly from the frame.
25. The eyewear of any of aspects 20-24, wherein the camera module comprises:
   a camera module housing including an aperture for the camera lens;
      and
   an image sensor disposed within the camera module housing.
26. The eyewear of any of aspects 20-25, further comprising:
   a camera battery;
   a memory to store electronic data related to the image sensor; and
   a wireless transceiver to transmit and receive the electronic data related to the image sensor.
27. The eyewear of aspect 26, wherein the camera battery and wireless transceiver are disposed inside the camera module housing.
28. The eyewear of aspect 26, wherein the camera battery and wireless transceiver are disposed inside a second housing.
29. The eyewear of aspect 28, wherein the second housing is supported solely by the eyewear lens.
30. The eyewear of aspect 8, wherein the second housing is supported by a goggle strap connected to the eyewear lens frame.
31. The eyewear of any of aspects 20-30, wherein the eyewear lens is a unitary shield lens.
32. The eyewear of any of aspects 20-31, wherein the camera module is detachably attached to the eyewear lens.
33. The eyewear of any of aspects 25-32, wherein at least a portion of the camera module housing is positioned on an interior portion of the eyewear lens.
34. The eyewear of aspect 33, wherein the camera module housing comprises a first portion positioned on an interior portion of the eyewear lens and a second portion positioned on an exterior portion of the eyewear lens.
35. The eyewear of aspect 34, wherein the first portion is detachably attached to the second portion.
36. The eyewear of any of aspects 25-35, wherein at least a portion of the camera module housing extends through an aperture in the eyewear lens.
37. The eyewear of aspect 36, wherein the aperture forms a channel in a top surface of the eyewear lens.
38. The eyewear of aspect 37, wherein an interior of the channel includes a protrusion to engage the camera module housing.
39. The eyewear of any of aspects 20-38, wherein the camera module is adjustable to alter a viewing angle of the camera module.
40. The eyewear of any of aspects 20-39, wherein the camera module captures a point of view of a wearer.
41. The eyewear of any of aspects 20-40, wherein the camera module is positioned at a top portion of the lens substantially centered on a wearer's brow.
42. A method for forming an eyewear lens assembly, comprising:
   connecting a camera module comprising a camera lens and an image sensor to an eyewear lens such that the camera module is supported exclusively by the eyewear lens.
43. The method of aspect 42, wherein the camera module captures a point of view of a wearer.
44. The method of any of aspects 42-43, wherein the connecting the camera module to the eyewear lens includes fixing the camera module to the eyewear lens.
45. The method of any of aspects 42-43, wherein the connecting the camera module to the eyewear lens includes detachably attaching the camera module to the eyewear lens.
46. The method of any of aspects 42-45, wherein the connecting the camera module to the eyewear lens includes positioning a portion of the camera module in an aperture in the eyewear lens.
47. The method of aspect 46, wherein the aperture forms a channel in a top surface of the eyewear lens.
48. The goggle of aspect 47, wherein an interior of the channel includes a protrusion to engage the camera module.
49. Eyewear comprising the eyewear lens assembly of any of aspects 1-19.
50. The eyewear of aspect 49, wherein the eyewear is a goggle.
51. The eyewear of aspect 49, wherein the eyewear is a helmet visor.

## Claims

1. A eyewear lens assembly for eyewear comprising:
an eyewear lens; and
a camera module comprising a camera lens, the camera module being connected to and supported solely by the eyewear lens.

2. The eyewear lens assembly of claim 1, wherein the camera module captures a point of view of a wearer.

3. The eyewear lens assembly of any preceding claim, wherein the camera module is positioned at a top portion of the lens substantially centered on the lens.

4. The eyewear lens assembly of any preceding claim, wherein the eyewear is a goggle.

5. The eyewear lens assembly of any preceding claim, wherein the camera module comprises:
a camera module housing including an aperture for the camera lens; and
an image sensor disposed within the camera module housing.

6. The eyewear lens assembly of claim 5, further comprising:
a camera battery;
a memory to store electronic data related to the image sensor; and
a wireless transceiver to transmit and receive the electronic data related to the image sensor.

7. The eyewear lens assembly of claim 6, wherein the camera battery and wireless transceiver are disposed inside the camera module housing.

8. The eyewear lens assembly of any preceding claim, wherein the eyewear lens is a unitary shield lens for eyewear.

9. The eyewear lens assembly of any preceding claim, wherein the camera module is detachably attached to the eyewear lens.

10. The eyewear lens assembly of claim 5, wherein at least a portion of the camera module housing is positioned on an interior portion of the eyewear lens.

11. The eyewear lens assembly of claim 5 or claim 10, wherein the camera module housing comprises a first portion positioned on an interior portion of the eyewear lens and a second portion positioned on an exterior portion of the eyewear lens.

12. The eyewear lens assembly of claim 11, wherein the first portion is detachably attached to the second portion.

13. The eyewear lens assembly of claim 5 or claim 10, wherein at least a portion of the camera module housing extends through an aperture in the eyewear lens.

14. The eyewear lens assembly of claim 13, wherein the aperture forms a channel in a top surface of the eyewear lens.

15. The eyewear lens assembly of claim 14, wherein an interior of the channel includes a protrusion to engage the camera module housing.

16. The eyewear lens assembly of any preceding claim, wherein the camera module is adjustable to alter a viewing angle of the camera lens.
